# FASCICULE DE BREVET EUROPEEN

(11) **EP 0 371 893 B1**
(45) Date de publication et mention de la délivrance du brevet: **31.03.1993**
(21) Numéro de dépôt: 89420466.8
(22) Date de dépôt: 24.11.1989
(51) Int. Cl.: B65H 45/18, B65H 45/30

(54) **Procédé et dispositif de pliage de bandes de matériaux textiles notamment de compresses**
Verfahren und Einrichtung zum Falzen von Textilienbahnen, insbesondere Kompressen
Method and device for folding textile webs, particularly compresses

(30) Priorité: 01.12.1988 FR 8816491
(43) Date de publication de la demande: 06.06.1990
(73) Titulaire: Marion, Louis, F-42230 Saint Victor Sur Loire (FR)
(72) Inventeur: Marion, Louis, F-42230 Saint Victor Sur Loire (FR)
(74) Mandataire: Dupuis, François

(56) Documents cités:
- EP-A- 0 056 353
- FR-A- 2 146 021
- FR-A- 2 279 551
- FR-A- 2 564 812
- GB-A- 1 400 805

## Description

L'invention concerne un procédé et un dispositif de pliage de bandes de matériaux textiles, notamment de compresses.

L'objet de l'invention se rattache au secteur technique des moyens de traitement de matériaux souples enroulés en bandes ou feuilles, et coupés à longueur, en vue de leur conditionnement.

On connait des machines destinées à réaliser en continu et automatiquement des compresses, cela en partant de bobines qui sont déroulées pour amener des bandes vers des moyens de coupe à longueur, puis des moyens de pliage transversal des bandes et enfin des moyens de conditionnement des bandes pliées.

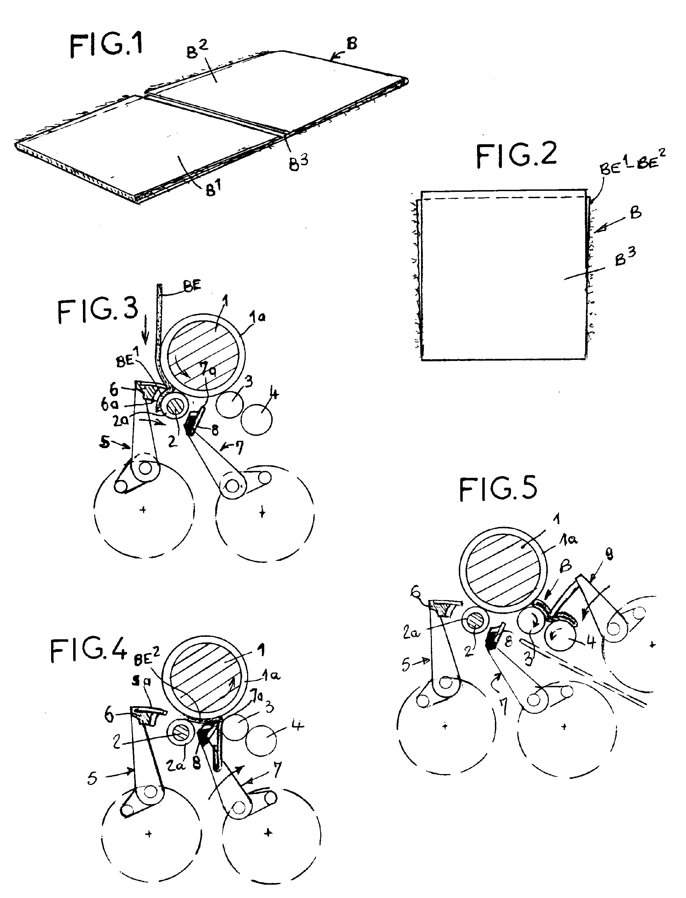

Lorsque les compresses sont pliées en quatre, comme c'est le cas dans une machine ayant fait l'objet d'un brevet EP-A-56 353 avec priorité francaise n° 81.00687 dont le déposant est également le titulaire, les parties extrêmes (B1-B2) de la bande (B) qui sont repliées successivement sur la partie centrale (B3) ont tendance à s'écarter transversalement sous l'effet d'écrasement des cylindres plieurs et à s'effilocher en présentant des fils en débordement de la largeur de la bande, comme on l'a illustré aux figures 1 et 2. On conçoit que ce phénomène nuit à une bonne présentation du produit et au conditionnement ultérieur, en particulier lorsque plusieurs compresses sont réunies dans un sachet ou dans un boîte.

Ces inconvénients sont résolus avec le procédé et le dispositif selon l'invention qui permettent de plier en quatre une compresse de manière nette, sans débordement ou effilochage de ses parties extrêmes, cela en continu dans un machine entre le poste de distribution et le poste de conditionnement.

Pour cela, et selon une première caractéristique, on effectue simultanément avec les premier et deuxième plis des deux parties d'extrémité de la bande coupée à longueur une retreinte transversale desdites parties afin qu'après la formation du troisième pli de la partie centrale de la bande et la superposition des deux plis extrêmes, lesdites parties d'extrémité soient en retrait de la largeur de la partie centrale.

Une autre caractéristique se trouve dans le fait que la retreinte transversale des parties d'extrémité de la bande coupée à longueur est obtenue par des contre-pièces rendues solidaires des premier et deuxième moyens de pliage et conformées pour obliger lesdites extrémités à pénétrer partiellement dans des rainures circulaires réalisées sur un cylindre d'entrainement et un rouleau de contre-appui entre lesquels passe la bande.

Selon un autre caractéristique les contre-pièces sont réalisées sous la forme d'un peigne fixé à la partie dentelée des moyens de pliage, et constitué par des saillies convexes alternées avec des dégagements.

Ces caractéristiques et d'autres encore ressortiront de la description qui suit.

Pour fixer l'objet de l'invention sans toutefois le limiter, dans les dessins annexés :

Les figures 1 et 2 illustrent respectivement par une vue en perspective et par une vue en plan une compresse pliée selon l'art antérieur.

Les figures 3, 4 et 5 sont des vues à caractère schématique montrant les pliages successifs de la compresse.

La figure 6 est une vue en coupe à plus grande échelle illustrant la formation de la rétreinte transversale des parties d'extrémité de la compresse.

La figure 7 est une vue en plan et en coupe considérée suivant la ligne 7-7 de la figure 6.

La figure 8 est une vue en perspective montrant une compresse réalisée selon l'invention avec ses parties d'extrémité repliées sur la partie centrale.

La figure 9 est une vue en plan illustrant la compresse de la figure 8 complètement pliée.

Afin de rendre plus concret l'objet de l'invention, on le décrit maintenant sous une forme non limitative de réalisation illustrée aux figures des dessins.

Les compresses à plier en quatre sont distribuées à partir de bobines, coupées à longueur et amenées vers les moyens de pliage par des organes bien connus associés entre eux sur un bâti général. La machine ainsi conçue permet de couper, de plier et de conditionner automatiquement et en continu les bandes nécessaires à la réalisation des compresses. Ces dispositions étant connues, on ne les a pas illustrés ; le domaine de l'invention commence au niveau du premier pliage de la bande (BE) comme on le voit à la figure 3.

La bande entrainée positivement par des rouleaux d'appel en amont, descend verticalement dans un couloir aménagé avec un système de soufflerie et d'aspiration combinés, passe entre des cylindres de coupe en position d'attente et arrive devant un cylindre (1) à gorges multiples (1a) dans lesquelles sont logées des palettes escamotables (non représentées) qui sont destinées à séparer les bandes coupées en amont.

En dessous du cylindre (1) sont placés en pression élastique des rouleaux (2) et (3), tandis qu'un troisième rouleau (4) est également appliqué contre le rouleau (3). Le rouleau (2) est aménagé avec des gorges multiples (2a) de même nature que les gorges (1a) du cylindre (1), tandis que les rouleaux (3) et (4) sont lisses.

Lorsque la partie d'extrémité (BE.1) de la bande se trouve contre le rouleau (2) un moyen de pliage (5) est actionné par tous moyens pour s'appliquer par sa partie transversale (5a) formée de dents, contre ladite partie (BE.1) qui est ainsi engagée et tenue entre le rouleau (2) et le cylindre (1) en vue de former le premier pli.

A ce moment, les cylindres de coupe en amont sont commandés pour sectionner la bande à la longueur désirée, puis par rotation du cylindre (1), la partie pliée de la bande passe entre le cylindre (1) et le rouleau (2), tandis que le moyen de pliage (5) recule légèrement puis avance de nouveau afin qu'une contre-pièce (6), rendue solidaire de la face inférieure de la partie (5a) et formant un peigne par ses saillies convexes (6a) alternées avec des dégagements (6b), s'appuie en pression contre le rouleau (2) en obligeant la partie d'extrémité (BE1) de la bande à pénétrer dans les gorges (2a) dudit rouleau (figures 3, 6 et 7). De cette manière, on comprend que cette partie de bande froncée par le peigne, subit un retrait transversal.

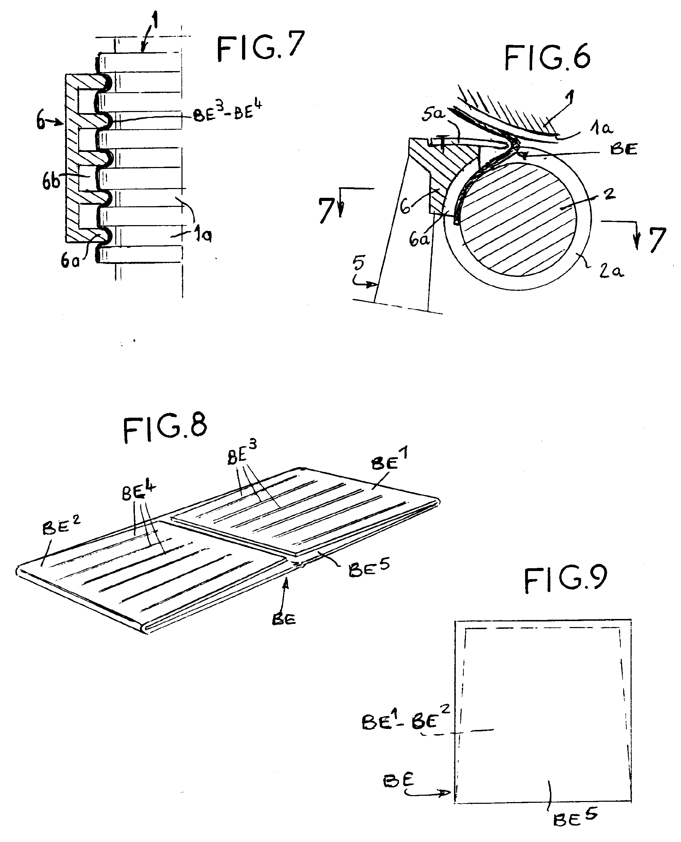

De la même façon, lorsque l'extrémité arrière (BE2) de la bande va être lachée par le cylindre (1) et le rouleau (2), un deuxième moyen de pliage (7) est actionné pour former le deuxième pli par sa partie transversale dentelée (7a) en engageant la bande entre le cylindre (1) et le rouleau (3) comme illustré à la figure 4. Comme précédemment, le moyen de pliage (7) présente une contre-pièce (8) fixée au-dessus de la partie dentelée (7a), afin qu'après léger recul puis avance, les saillies convexes (7b) du peigne de la contre-pièce forment des fronces de rétraction sur la partie (BE2) de la bande pendant son défilement.

La bande se présente alors avec ses extrémités (BE1) et (BE2), au-dessus des rouleaux (3) et (4) pour former le troisième pli central, à l'aide d'un troisième moyen de pliage (9) classique (figure 5), puis la bande est évacuée en direction du poste de conditionnement.

Comme on le voit bien aux figures 8 et 9, la compresse ainsi réalisée présente des parties d'extrémité (BE1) et (BE2) avec des fronces (BE3) et (BE4) leur donnant une largeur inférieure à celle de la partie centrale (BE5), ce qui permet une fois formé le troisième pli, d'obtenir une compresse aux bords nets et non effilochés facilitant le conditionnement ultérieur et améliorant la présentation.

## Revendications

1. Procédé de pliage de bandes de matériaux textiles, notamment de compresses pliées en quatre, caractérisé en ce qu'on effectue simultanément avec les premier et deuxièmes plis des deux parties d'extrémité (BE1) et (BE2) de la bande (BE) coupée à la longueur, une retreinte transversale desdites parties afin qu'après formation du troisième pli de la partie centrale (BE5) de la bande et superposition des deux plis extrêmes, lesdites parties d'extrémité soient en retrait de la largeur de la partie centrale.

2. Dispositif de pliage mis en oeuvre selon le procédé de la revendication 1, caractérisé en ce que la retreinte transversale des parties d'extrémité (BE1) et (BE2) de la bande (BE) coupée à la longueur, est obtenue automatiquement pendant le défilement de ladite bande, par des contre-pièces (6) et (8) rendues solidaires des premier et deuxième moyens de pliage et conformées pour obliger lesdites extrémités de bandes à pénétrer partiellement dans des rainures circulaires (1a) et (2a) réalisées sur un cylindre (1) d'entrainement de la bande et un rouleau (2) maintenu en appui élastique contre le cylindre, et entre lesquels passe la bande.

3. Dispositif de pliage selon la revendication 2, caractérisé en ce que les contre-pièces (6) et (8) sont réalisées sous la forme d'un peigne fixé à la partie transversale dentelée (5a) et (7a) des moyens de pliage, et constitué par des saillies convexes (6a) et (7a) alternées avec des dégagements (66).

4. Dispositif de pliage selon l'une quelconque des revendications 2 et 3, caractérisé en ce que la contre-pièces (6) est fixée au-dessous de la partie transversale dentelée (5a) du moyen de pliage (5) pour agir sur la partie d'extrémité (BE1) de la bande appliquée sur le rouleau (2) pendant son défilement.

5. Dispositif de pliage selon l'une quelconque des revendications 2 et 3, caractérisé en ce que la contre-pièce (8) est fixée au-dessus de la partie transversale dentelée (7a) du moyen de pliage (7) pour agir sur la partie d'extrémité (BE2) de la bande appliquée contre le cylindre (1) d'entrainement pendant son défilement.

## Patentansprüche

1. Verfahren zum Verfalten von Bahnen aus gewebten Materialien, insbesondere von vierfachgefalteten Verbandsbinden, dadurch gekennzeichnet, dass zur gleichen Zeit mit der ersten und der zweiten Faltbildung der beiden Endteile (BR1) und (BE2) der zu der erforderlichen Länge zerschnittenen Bahn (BE) ein Quereinschrumpfen der besagten Teile durchgeführt wird, damit die genannten Endteile, nach der dritten Faltbidlung des mittigen Teils (BE5) der Bahn und nach dem Aufeinanderliegen der zweien äussersten Faltbildungen abstehend zu der Breite der Bahn zu liegen kommen.

2. Verfaltenvorrichtung zum Bewerkstelligen gemäss dem Verfahren nach Aspruch 1, dadurch gekennzeichnet, dass das Querverschrumpfen der Endteile (BEI) und (BE2) der zu der erforderlichen Länge zerschnittenen Bahn (BE) während"der Vorbeiverschiebung der genannten Bahn automatisch mittels Gegenstücke (6) und (8) erreicht wird, die mit dem ersten und dem zweiten Verfaltungsmittel festgemacht werden und so eingerichtet sind, dass diese Gegenstücke die genannten Bahnenden zum teilweisen Eindringen in die auf einem Zylinder (1) zum Antrieb der Bahn und auf einer in federnder Abstützung gegen den basagten Zylinder gehalteten Rolle (2) vorgesehenen kreisförmigen Nuten (1a) und (2a) zwingen, wobei die Bahn zwischem dem Ylinder und der Rolle durchläuft.

3. Verfaltenvorrichtung nach Anspruch 2, dadurch gekennzeichnet, dass die Gegenstücke (6) und (8) als ein Rietblatt ausgestaltet sind, das auf dem verzahnten Querteil (5a) und (7a) der Verfaltungsmittel befestigt wird, und aus mit Freiabständen (66) abwechselnden, konvexförmigen Vorsprüngen (6a) und (7a) besteht.

4. Verfaltenvorrichtung nach einem der Ansprüche 2 und 3, dadurch gekennzeichnet, dass das Gegenstück (6) unterhalb des verzahnten Querteils (5a) des Verfaltungsmittels (5) befestigt ist, um auf den Endeteil (BE1) der auf die Rolle (2) aufgelegten Bahn während der Vorbeibewegung dieser Bahn zu wirken.

5. Verfaltenvorrichtung nach einem der Ansprüche 2 und 3, dadurch gekennzeichnet, dass das Gegenstück (8) oberhalb des versahnten Querteils (7a) des Verfaltungsmittels (7) befestigt ist, um auf den Endeteil (BE2) der auf den Antriebszylinder (1) aufgelegten Bahn während der Vorbeibewegung dieser Bahn zu wirken.

## Claims

1. Folding method for webs of textile material, in particular compress pads folded up four times, characterised in that together with the first and the second folding up of the two end portions (BE1) and (BE2)of the web (B2) cut to length a transverse straitening is simultaneously applied on said portions so that after the formation of the third fold of the central portion (BE5) of the web and the superimposition of the two extreme folds, the end portions will be receding relative to the width of the central portion.

2. Folding device to be worked out in accordance wuth the method of Claim 1, characterised in that the transverse straitening of the end portions (BE1) and (BE2) of the web (BE) cut at length is performed automatically during the onward movement of said web, by means of the counter-pieces (6) and (8) which are made depending on the first and the second folding means, and are devised to cause said web ends to penetrate partially into the circular grooves (1a) and (2a) provided on a cylinder (1) driving the web and on a roller (2) held in elastical abutment against the cylinder, the web being passed between the cylinder and the roller.

3. Folding device according to Claim 2, characterised in that the counter-pieces (6) and (8) are made in the form of a reed secured to the serrated transverse portion (5a) and (7a) of the folding means, and consisting of convex projections (6a) and (7a) in alternating arrangement with clearances (66).

4. Folding device according to any one of Claims 2 and 3, characterised in that the counter-piece (6) is secured beneath the serrated transverse portion (5a) of the folding means (5) in order to operate on the end portion (BE1) of the web laid on the roller (2) during the onward movement of said web.

5. Folging device according to any one of Claims 2 and 3, characterised in that the counter-piece (8) is secured above the serrated transverse portion (7a) of the folding means (7) in order to operate on the end portion (BE2) of the web laid on against the driving cylinder (1) during the onward movement of said web.
